# EUROPÄISCHE PATENTANMELDUNG

(11) **EP 2 269 655 A2**
(43) Veröffentlichungstag der Anmeldung: **05.01.2011**
(21) Anmeldenummer: 10011196.2
(22) Anmeldetag: 28.02.2003
(51) Int. Cl.: A61K 47/48

(54) **Kopplung niedermolekularer Substanzen an hydroxyalkylstärke**

(30) Priorität: 06.03.2002 DE 10209822
(62) Teilanmeldung aus: 03722333.6
(71) Anmelder: Fresenius Kabi Deutschland GmbH, 61352 Bad Homburg (DE)
(72) Erfinder: Orlando, Michele, 35394 Giessen (DE); Hemberger, Jürgen, 35741 Aschaffenburg (DE); Sommermeyer, Klaus, 61191 Rosbach v.d.H. (DE); Eichner, Wolfram, 35510 Butzbach (DE); Frie, Sven, 1967 Bramois (CH); Lutterbeck, Katharina, 61169 Friedberg (DE); Jungheinrich, Cornelius, 61350 Bad Homburg (DE); Scharpf, Roland, 63691 Ranstadt (DE)
(74) Vertreter: Altmann, Andreas

(57) **Zusammenfassung**

Die vorliegende Erfindung betrifft die Kopplung von niedermolekularen Substanzen an ein von Stärke abgeleitetes modifiziertes Polysaccharid, wobei die bindende Wechselwirkung zwischen dem modifizierten Polysaccharid und der niedermolekularen Substanz auf einer kovalenten Bindung beruht, welche das Ergebnis einer Kopplungsreaktion zwischen der endständigen Aldehydgruppe oder einer aus dieser Aldehydgruppe durch chemische Umsetzung hervorgegangenen funktionellen Gruppe des modifizierten Polysaccharidmoleküls und einer mit dieser Aldehydgruppe oder daraus hervorgegangenen funktionellen Gruppe des Polysaccharidmoleküls reaktionsfähigen funktionellen Gruppe der niedermolekularen Substanz ist, wobei die bei der Kopplungsreaktion unmittelbar resultierende Bindung gegebenenfalls durch eine weitere Reaktion zur obengenannten kovalenten Bindung modifiziert sein kann.
Die Erfindung betrifft ferner pharmazeutische Zusammensetzungen, welche die bei der Kopplung gebildeten Konjugate umfassen, und die Verwendung dieser Konjugate und Zusammensetzungen zur prophylaktischen oder therapeutischen Behandlung des menschlichen oder tierischen Körpers.

## Beschreibung

Es gibt eine Vielzahl niedermolekularer Substanzen von kommerziellem Interesse, insbesondere Arzneiwirkstoffe und Pflanzenschutzmittel, deren Einsatz durch unbefriedigende Löslichkeitseigenschaften im wässrigen Milieu und/oder geringe Verweilzeit im Körper eingeschränkt oder sogar verhindert wird. So werden beispielsweise kleine Arzneimittelmoleküle durch die glomeruläre Filtration in der Niere (Ausschlussgrenze etwa 70 kD) häufig zu schnell wieder aus dem Kreislauf entfernt, so dass eine ständige kostspielige und für den Patienten unbequeme Nachversorgung mit diesem Medikament, z. B. durch häufig wiederholte Verabreichungen oder Infusion, erforderlich ist.

Um diesen Nachteil zu umgehen, werden in einigen Fällen schwerlösliche Arzneiwirkstoffe als öliger Bolus verabreicht, der an der Injektionsstelle häufig schmerzhafte Ablagerungen bildet. Darüber hinaus ist der Einsatz solcher schwerlöslicher Medikamente oft mit toxischen Nebenwirkungen aufgrund ihrer Ablagerung in Organen wie Leber und/oder Niere verbunden. Solche unerwünschten Nebenwirkungen haben wiederum zur Folge, dass der *in vivo* einsetzbare Konzentrationsbereich des Wirkstoffes stark eingeschränkt ist.

Ein in jüngerer Zeit verfolgter Ansatz zur Behebung der geschilderten Probleme besteht in der Kopplung solcher problematischer Substanzen an gut lösliche biokompatible Polymeren, wie z. B. Polyethylenglycol und Dextran. Durch die Kopplung lässt sich einerseits das Molekulargewicht über den Schwellenwert von 70 kD hinaus erhöhen, so dass die Plasma-Verweilzeit kleinerer Moleküle drastisch gesteigert werden kann, andererseits kann durch den hydrophilen Polymeranteil die Löslichkeit im wässrigen Milieu verbessert werden.

Bisher wurden die meisten Modifizierungen mit Polyethylenglycol oder Dextran durchgeführt, wobei PEG generell bevorzugt wird, da es einfachere Produkte ergibt. Dextran-Konjugate zeigen jedoch oft hohe Allergenität, eine geringe metabolische Stabilität und in vielen Fällen geringe Ausbeuten bei den Kopplungsreaktionen. Beim Einsatz von PEG-Konjugaten wurden ebenfalls über unangenehme bis gefährliche Nebenwirkungen wie Juckreiz, Überempfindlichkeitsreaktionen und Pankreatitis berichtet. Ferner ist öfter die biologische Aktivität der Wirkstoffe nach der PEG-Kopplung zum Teil stark erniedrigt. Zudem ist der Metabolismus der Abbauprodukte von PEG-Konjugaten noch weitgehend unbekannt und stellt möglicherweise ein Gesundheitsrisiko dar.

Es besteht somit nach wie vor Bedarf an physiologisch gut verträglichen Alternativen zu Dextran- oder PEG-Konjugaten, mit denen die Löslichkeit von schlecht löslichen niedermolekularen Substanzen verbessert und/oder die Verweildauer von niedermolekularen Substanzen im Plasma erhöht werden kann, wodurch verbesserte pharmakodynamischen Eigenschaften des Wirkmoleküls erreicht werden.

Aufgabe der Erfindung ist es daher, solche Alternativen bereitzustellen und einfache und effiziente Verfahren zur Herstellung solcher alternativer Konjugate zu entwickeln.

Überraschend wurde gefunden, dass sich diese Aufgabe durch Hydroxyalkylstärke-Konjugate lösen lässt, welche dadurch gekennzeichnet sind, dass die bindende Wechselwirkung zwischen dem Hydroxyalkylstärkemolekül und der niedermolekularen Substanz auf einer kovalenten Bindung beruht, welche das Ergebnis einer Kopplungsreaktion zwischen der endständigen Aldehydgruppe oder einer aus dieser Aldehydgruppe durch chemische Umsetzung hervorgegangenen funktionellen Gruppe des Hydroxyalkylstärkemoleküls und einer mit dieser Aldehydgruppe oder daraus hervorgegangenen funktionellen Gruppe des Hydroxyalkylstärkemoleküls reaktionsfähigen funktionellen Gruppe der niedermolekularen Substanz ist, wobei die bei der Kopplungsreaktion unmittelbar resultierende Bindung gegebenenfalls durch eine weitere Reaktion zur obengenannten kovalenten Bindung modifiziert sein kann.

Die Erfindung umfasst ferner pharmazeutische Zusammensetzungen, welche diese Konjugate enthalten, sowie die Verwendung dieser Konjugate und Zusammensetzungen zur prophylaktischen oder therapeutischen Behandlung des menschlichen oder tierischen Körpers sowie Verfahren zur Herstellung dieser Konjugate und Zusammensetzungen.

Die erfindungsgemäß eingesetzte Hydroxyalkylstärke (HAS) kann nach einem bekannten Verfahren, z. B. Hydroxyalkylierung von Stärke an der C₂- und/oder C₆-Position der Anhydroglucoseeinheiten mit Alkylenoxid oder 2-Chloralkanol, z. B. 2-Chlorethanol, (siehe z. B. US 5,218,108 für die Hydroxyethylierung von Stärke), mit verschiedenen gewünschten Molekulargewichtsbereichen und Substitutionsgraden hergestellt werden. Es können auch beliebige der im Handel erhältlichen Präparationen eingesetzt werden. Die Definition der Alkylgruppierung in "Hydroxyalkystärke", wie hier verwendet, schließt Methyl, Ethyl, Isopropyl und n-Propyl ein, wobei Ethyl besonders bevorzugt ist. Ein wesentlicher Vorteil der Hydroxyethylstärke (HES) ist es, dass diese als biokompatibler Plasmaexpander bereits behördlich zugelassen ist und in großem Stil klinisch eingesetzt wird.

Das mittlere Molekulargewicht der Hydroxalkylstärke kann im Bereich von etwa 3 kD bis mehreren Millionen Dalton, vorzugsweise etwa 10 kD bis etwa 2000 kD, bevorzugter im Bereich von etwa 70 kD bis etwa 1000 kD, besonders bevorzugt bei etwa 130 kD, liegen. Zur Erhöhung der Verweilzeit der niedermolekularen Substanz im Organismus wird das mittlere Molekulargewicht der Hydroxyalkylstärke vorzugsweise so gewählt, das der glomeruläre Schwellenwert von 70 kD bei den Konjugaten überschritten wird. Der Substitutionsgrad (Verhältnis der Anzahl modifizierter Anhydroglucoseeinheiten zur Anzahl der gesamten Anhydroglucoseeinheiten) kann ebenfalls variieren und wird häufig im Bereich von etwa 0,2 bis 0,8, vorzugsweise etwa 0,3 bis 0,7, noch bevorzugter etwa 0,5, liegen. (Anmerkung: Die Zahlen beziehen sich auf den "Degree of Substitution", der zwischen 0 und 1 liegt). Das Verhältnis von C₂- zu C₆-Substitution liegt gewöhnlich im Bereich von 4 bis 16, vorzugsweise im Bereich von 8 bis 12.

Diese Parameter lassen sich nach bekannten Verfahren einstellen. Erfahrungen mit der Verwendung von Hydroxyethylstärke als Blutersatzstoff haben gezeigt, dass die Verweilzeit von HES im Plasma vom Molekulargewicht und dem Substitutionsgrad und Substitutionstyp (C₂-Substitution oder C₆-Substitution) abhängt, wobei ein höheres Molekulargewicht, ein höherer Substitutionsgrad und ein höherer Anteil der C₂-Substitution die Verweilzeit erhöht.

Diese Beziehungen gelten auch für die erfindungsgemäßen Konjugate von Hydroxyalkylstärke und niedermolekularen Substanzen, so dass die Verweilzeit eines bestimmten Konjugats im Plasma über den Polysaccharidanteil einstellbar ist.

Wie bereits erwähnt, ist die an der Kopplungsreaktion beteiligte funktionelle Gruppe des Hydroxyalkylstärkemoleküls die endständige Aldehydgruppe oder eine daraus durch chemische Umsetzung hervorgegangene Funktionalität.

Ein Beispiel für eine solche chemische Umsetzung ist die selektive Oxidation dieser Aldehydgruppe mit einem geeigneten Oxidationsmittel, wie z. B. lod, Brom oder einigen Metallionen, oder auch mittels elektrochemischer Oxidation zu einer Carboxylgruppe oder aktivierten Carboxylgruppe, z. B. einem Ester, Lacton, Amid, wobei die Carboxylgruppe gegebenenfalls in einer zweiten Reaktion in das aktivierte Derivat überführt wird. Diese Carboxylgruppe oder aktivierte Carboxylgruppe kann dann mit einer primären Amino- oder Thiolgruppe der niedermolekularen Substanz unter Ausbildung einer Amidbindung oder Thioesterbindung gekoppelt werden. Eine weitere Möglichkeit ist die Kopplung mit einer Hydroxyl-Funktion der niedermolekularen Substanz unter Esterbildung.

Ein erfindungsgemäßes Konjugat kann aber auch dadurch erhalten werden, dass die niedermolekulare Substanz zur Einführung einer gewünschten funktionellen Gruppe mit einem geeigneten physiologisch verträglichen bifunktionellen Linkermolekül umgesetzt wird. Die verbleibende reaktionsfähige Gruppe des angekoppelten Linkermoleküls wird für die Zwecke der vorliegenden Erfindung ebenfalls als "reaktionsfähige funktionelle Gruppe der niedermolekularen Substanz" betrachtet.

Geeignete Linkermoleküle enthalten an einem Ende eine Gruppierung, die mit einer reaktionsfähigen funktionellen Gruppe der niedermolekularen Substanz, z. B. einer Amino-, Thiol-, Carboxyl- oder Hydroxy-Gruppe, eine kovalente Bindung eingehen kann, und am anderen Ende eine Gruppierung, die mit der endständigen Aldehydgruppe oder einer daraus durch chemische Umsetzung hervorgegangenen funktionellen Gruppe, z. B. einer Carboxylgruppe, aktivierten Carboxylgruppe, Amino- oder Thiolgruppe, ebenfalls eine kovalente Bindung eingehen kann.

Zwischen den beiden funktionellen Gruppen des Linkermoleküls befindet sich ein biologisch verträgliches Brückenmolekül geeigneter Länge, z. B. eine Gruppierung, die sich von einem Alkan ableitet, eine (Oligo)alkylenglycolgruppierung oder eine andere geeignete Oligomergruppierung. Bevorzugte Gruppierungen, die mit Aminogruppen reagieren können, sind z. B. N-Hydroxysuccinimidester, Sulfo-N-hydroxysuccinimid-ester, lmidoester oder andere aktivierte Carboxylgruppen; bevorzugte Gruppierungen, die mit Thiolgruppen reagieren können, sind z.B. Maleimid- und Carboxylgruppen; bevorzugte Gruppierungen, die mit Aldehyd- oder Carboxyl-gruppen reagieren können, sind z. B. Amino- oder Thiolgruppen.

Beispielhafte Linkermoleküle zur Verknüpfung von SH- und NH-Funktionen sind:

| | |
|---|---|
| AMAS | (N-α(Maleimidoacetoxy)succinimidester) |
| BMPS | (N-β(Maleimidopropyloxy)succinimidester) |
| GMBS | (N-γ(Maleimidobutyryloxy)succinimidester) |
| EMCS | (N-ε(Maleimidocaproyloxy)succinimidester) |
| MBS | (m-(Maleimidobenzoyl)-N-hydroxysuccinimidester) |
| SMCC | (Succinimidyl-4-(N-maleimidomethyl)cyclohexan-1-carboxylat) |
| SMPB | (Succinimidyl-4-(p-maleimidophenyl)butyrat) |
| SPDP | (Succinimidyl-3-(2-pyridyldithio)proprionat) |
| Sulfo-GMBS | (N-γ(Maleimidobutyryloxy)suifosuccinimidester) |
| Sulfo-EMCS | (N-ε(Maleimidocaproyloxy)sulfosuccinimidester). |

Beispielhafte Linkermoleküle zur Verknüpfung von SH- und SH-Funktionen sind:

| | |
|---|---|
| BMB | (1.4-Bis-maleimidobutan) |
| BMDB | (1.4-Bis-maleimido-2.3-dihydroxybutan) |
| BMH | (Bis-maleimidohexan) |
| BMOE | (Bis-maleimidoethan) |
| DTME | (Dithio-bis-maleimidoethan) |
| HBVS | (1.6-Hexan-bis-vinylsulfon) |
| BM(PEO)₃ | (1.8-Bis-maleimidotriethylenglycol) |
| BM(PEO)₄ | (1.11-Bis-maleimidotetraethylenglycol). |

Beispielhafte Linkermoleküle zu Verknüpfung von NH- und NH-Funktionen sind:

| | |
|---|---|
| BSOCOES | (Bis-(2-(succinimidyloxycarbonyloxy)ethyl)sulfon |
| BS³ | (Bis-(sulfosuccinimidyl)suberat) |
| DFDNB | (1.5-Difluor-2.4-dinitrobenzol) |
| DMA | (Dimethyladipimidat HCl)) |
| DSG | (Disuccinimidylglutarat) |
| DSS | (Disuccinimidylsuberat) |
| EGS | (Ethylenglycol-bis-(succinimidylsuccinat). |

Beispielhafte Linkermoleküle zur Verknüpfung von SH- und CHO-Funktionen sind:

| | |
|---|---|
| BMPH | (N-(β-Maleimidopropionsäure)hydrazid TFA) |
| EMCA | (N-(ε-Maleimidocapronsäure)hydrazid) |
| KMUH | (N-(κ-Maleimidoundecansäure)hydrazid) |
| M₂C₂H | (4-(N-Maleimidomethyl)cyclohexan-1-carboxylhydrazid HCl) |
| MPBH | (4-(4-N-Maleimidophenyl)buttersäurehydrazid HCl) |
| PDPH | (3-(2-Pyridyldithio)propionylhydrazid). |

Ein beispielhaftes Linkermolekül zur Verknüpfung von SH- und OH-Funktionen ist PMPI (N-(p-Maleimidophenyl)isocyanat).

Beispielhafte Linkermoleküle zur Überführung einer SH-Funktion in eine COOH-Funktion sind:

| | |
|---|---|
| BMPA | (N-β-Maleimidopropionsäure) |
| EMCH | (N-β-Maleimidocapronsäure) |
| KMUA | (N-κ-Maleimidoundecansäure). |

Beispielhafte Linkermoleküle zur Überführung einer NH-Funktion in eine COOH-Funktion sind MSA (Methyl-N-succinimidyladipat) oder längerkettige Homologe davon oder entsprechende Derivate von Ethylenglycol.

Beispielhafte Linkermoleküle zur Überführung einer COOH-Funktion in eine NH-Funktion sind DAB (1.4-Diaminobutan) oder längerkettige Homologe davon oder entsprechende Derivate von Ethylenglycol.

Ein beispielhaftes Linkermolekül, das mit einer Aminogruppe eines Moleküls reagiert und eine geschützte Aminogruppe in größerem Abstand von diesem Molekül zur Vermeidung einer sterischen Hinderung bereitstellt, ist TFCS (N-ε(Trifluoracetylcaproyloxy)succinimidester).

Weitere geeignete Linkermoleküle sind Fachleuten bekannt und im Handel erhältlich oder können je nach Bedarf und in Abhängigkeit von den vorhandenen und gewünschten funktionellen Gruppen der HAS und der anzukoppelnden niedermolekularen Substanzen entworfen und nach bekannten Verfahren hergestellt werden.

In einem besonders bevorzugten Herstellungsverfahren wird die endständige Aldehydgruppe der HAS mit einem molaren Überschuss an lod, vorzugsweise in einem Molverhältnis von lod zu HAS von 2:1 bis 20:1, besonders bevorzugt etwa 5:1 bis 6:1, in wässriger basischer Lösung selektiv oxidiert. Nach dem in Beispiel 1 beschriebenen optimierten Verfahren wird zunächst eine Menge an Hydroxyalkylstärke in warmem destilliertem Wasser gelöst und etwas weniger als 1 Moläquivalent wässriger lodlösung, vorzugsweise in einer Konzentration von etwa 0,05-0,5 N, besonders bevorzugt etwa 0,1 N, zugegeben. Danach wird eine wässrige NaOH-Lösung in einer molaren Konzentration, die etwa das 5-15fache, vorzugsweise etwa das 10fache, der lodlösung beträgt, langsam tropfenweise in Abständen von mehreren Minuten der Reaktionslösung zugegeben, bis die Lösung nach der Zugabe beginnt, wieder klar zu werden. Es wird erneut etwas weniger als 1 Moläquivalent der obigen wäßrigen lodlösung der Reaktionslösung zugegeben, das Zutropfen der NaOH-Lösung wieder aufgenommen und die Zugabe von lod und NaOH werden solange wiederholt, bis etwa 5,5-6 Moläquivalente lodlösung und 11-12 Moläquivalente NaOH-Lösung, bezogen auf die Hydroxyalkylstärke, zugegeben worden sind. Dann wird die Reaktion abgebrochen, die Reaktionslösung entsalzt, z.B. durch Dialyse oder Ultrafiltration, einer Kationenaustauschchromatographie unterworfen und das Reaktionsprodukt durch Lyophilisierung gewonnen. Bei diesem Verfahren werden unabhängig vom Molekulargewicht der HAS fast quantitative Ausbeuten erzielt.

In einer weiteren besonders bevorzugten Ausführungsform erfolgt die selektive Oxidation mit alkalischen stabilisierten Lösungen von Metallionen, z. B. Cu⁺⁺ oder Ag⁺, ebenfalls in etwa quantitativer Ausbeute (Beispiel 2). Vorzugsweise wird dabei ein etwa 3-10facher molarer Überschuss des Oxidationsmittels eingesetzt.

Anschließend wird die gebildete selektiv oxidierte Hydroxyalkystärke in einem geeigneten organischen Lösungsmittel mit einer primären Aminogruppe der gewünschten niedermolekularen Substanz unter Ausbildung einer Amidbindung umgesetzt. Bevorzugte Lösungsmittel wurden aus der Gruppe der polarnichtprotischen Lösungsmittel ausgewählt, besonders bevorzugt wurde Dimethylsulfoxid (DMSO) verwendet. Im Gegensatz zu gängigen, in der Literatur beschriebenen Verfahren für ähnliche Kopplungsreaktionen wurde dabei überraschenderweise gefunden, dass der Einsatz ansonsten obligatorischer Aktivatoren wie Carbodiimide und Triazole nicht erforderlich ist. Die Kopplung von selektiv oxidierter Hydroxethylstärke (ox-HES) an verschiedene Modellverbindungen (siehe Beispiele) ging auch in Abwesenheit eines Aktivators glatt vonstatten.

Vorzugsweise finden die Kopplungsreaktionen jedoch in Gegenwart eines Carbodiimids, mehr bevorzugt in der Gegenwart von DCC (Dicyclohexyldicarbodiimid), am meisten bevorzugt in der Gegenwart von EDC (1-(3-Dimethylaminopropyl)-3-ethylcarbodiimid), statt.

Die reaktive Gruppe des Hydroxyalkylstärkemoleküls kann auch eine durch chemische Umsetzung der endständigen Aldehydgruppe entstandene Amin- oder Thiolgruppe sein. Beispielsweise kann eine reduktive Aminierung der Aldehydgruppe durch Reaktion mit Ammoniak in Gegenwart von Wasserstoff und einem Katalysator oder in Gegenwart von Natriumcyanoborhydrid durchgeführt werden. Die entstandene Amin- oder Thiolgruppe kann dann mit einer freien Carboxylgruppe oder Aldehydgruppe der niedermolekularen Substanz reagieren. Dabei entstehen zunächst Amid- oder Thioesterbindungen bzw. Schiff sche Basen, die gegebenenfalls durch eine weitere Reaktion modifiziert werden können.

Ferner kann die endständige Aldehydgruppe des Hydroxyalkylstärkemoleküls oder eine daraus durch chemische Umsetzung hervorgegangene funktionelle Gruppe auch mit einem geeigneten physiologisch verträglichen, bifunktionellen Linkermolekül umgesetzt werden. In diesem Falle ist für die Kopplungsreaktion die "aus der endständigen Aldehydgruppe des Hydroxyalkylstärkemoleküls durch chemische Umsetzung hervorgegangene funktionelle Gruppe" die verbleibende reaktionsfähige funktionelle Gruppe des bifunktionellen Linkermoleküls, mit dem die endständige Aldehydgruppe oder die daraus hervorgegangene funktionelle Gruppe umgesetzt wurde. Auf diese Weise kann die endständige Aldehydgruppe ebenfalls in eine gewünschte funktionelle Gruppe überführt werden.

Geeignete Linkermoleküle enthalten an einem Ende eine Gruppierung, die mit der endständigen Aldehydgruppe oder einer daraus durch chemische Umsetzung hervorgegangenen funktionellen Gruppe, z. B. einer Carboxylgruppe, aktivierten Carboxylgruppe, Amino- oder Thiolgruppe, eine kovalente Bindung eingehen kann, und am anderen Ende eine Gruppierung, die mit einer reaktionsfähigen funktionellen Gruppe der niedermolekularen Substanz, z. B. einer Amino-, Thiol-, Carboxyl- oder OH-Gruppe, vorzugsweise Aryl-OH-Gruppe, eine kovalente Bindung eingehen kann. Zwischen den beiden funktionellen Gruppen des Linkermoleküls befindet sich ein biologisch verträgliches Brückenmolekül geeigneter Länge, z. B. eine Gruppierung, die sich von einem Alkan ableitet, eine (Oligo)alkylenglycolgruppierung oder eine andere geeignete Oligomergruppierung. Bevorzugte Gruppierungen, die mit Aminogruppen reagieren können, sind z.B. N-Hydroxysuccinimidester, Sulfo-N-hydroxysuccinimidester, Imidoester oder andere aktivierte Carboxylgruppen; bevorzugte Gruppierungen, die mit Thiolgruppen reagieren können, sind z.B. Maleimid- und Carboxylgruppen; bevorzugte Gruppierungen, die mit Aldehyd- oder Carboxylgruppen reagieren können, sind z.B. Amino- oder Thiolgruppen.

Eine Reihe von speziellen, nicht beschränkenden Beispielen für geeignete Linkermoleküle wurden bereits oben unter Bezug auf die Konjugation von Linkermolekülen an niedermolekulare Substanzen angegeben.

Bei einem alternativen erfindungsgemäßen Kopplungsverfahren der vorliegenden Erfindung wird die endständige Aldehydgruppe der Hydroxyalkylstärke (HAS) direkt mit einer primären Aminogruppe der niedermolekularen Substanz bzw. eines an diese Substanz gekoppelten Linkermoleküls unter Bildung einer Schiff'schen Base umgesetzt. Anschliessend oder parallel dazu wird die gebildete Schiff'sche Base durch Umsetzung mit einem geeigneten Reduktionsmittel zum Amin reduziert, wodurch eine im wässrigen Milieu stabile Bindung zwischen niedermolekularer Substanz und HAS entsteht.

Bevorzugte Reduktionsmittel sind Natriumborhydrid, Natriumcyanoborhydrid, organische Borkomplexe, z.B. ein 4-(Dimethylamino)pyridin-Borkomplex, N-Ethyldiisopropylamin-Borkomplex, N-Ethylmorpholin-Borkomplex, N-Methylmorpholin-Borkomplex, N-Phenylmorpholin-Borkomplex, Lutidin-Borkomplex, Triethylamin-Borkomplex, Trimethylamin-Borkomplex; geeignete stereoselektive Reduktionsmittel sind beispielsweise Natriumtriacetatborhydrid, Natriumtriethylborhydrid, Natriumtrimethoxyborhydrid, Kalium-tri-sec-butylborhydrid (K-Selectride), Natrium-tri-sec-butylborhydrid (N-Selectride), Lithium-tri-sec-butylborhydrid (L-Selectride),Kaliumtriamylborhydrid (KS-Selectride) und Lithiumtriamylborhydrid (LS-Selectride).

Die Kopplungsreaktion von HAS oder oxidierter HAS an eine niedermolekulare Substanz wird aufgrund der zu erwartenden schlechten Wasserlöslichkeit der Substanz und der mangelnden Stabilität des Lactons in wässrigem Milieu vorzugsweise in einem organischen Lösungsmittel, bevorzugter einem polaren, nicht-protischen Lösungsmittel, in dem die HAS und vorzugsweise auch die niedermolekulare Substanz löslich ist, durchgeführt. Geeignete Lösungsmittel für HAS sind z. B. DMSO, Glycol, Diglycol, Triglycol sowie N-Methylpyn-olidon. Es ist auch möglich, Mischungen von DMSO mit anderen Lösungsmitteln einzusetzen, falls die niedermolekulare Substanz nicht in DMSO oder einem anderen bevorzugten Lösungsmittel für HAS löslich ist. Die Reaktion kann manchmal aber auch vorteilhaft in heterogener Phase durchgeführt werden.

Das Molverhältnis von HAS zu niedermolekularer Substanz bei der Kopplungsreaktion beträgt gewöhnlich etwa 20:1 bis 1:1, vorzugsweise etwa 5:1 bis 1:1.

Die Kopplungsausbeuten, bezogen auf die niedermolekulare Substanz, liegen in der Regel bei mehr als 40%, häufig mehr als 60% und nicht selten mehr als 80% (vgl. Beispiele).

Bei der zu koppelnden niedermolekularen Substanz handelt es sich vorzugsweise um einen Arzneiwirkstoff, dessen Löslichkeit in wässrigem Milieu und/oder dessen Bioverfügbarkeit, Stabilität und Verweilzeit im Körper erhöht werden soll. Der Begriff "niedermolekulare Substanz" soll auch Peptide bis zu etwa 50 Aminosäuren einschließen. Vorzugsweise ist der Arzneiwirkstoff aus der Gruppe aus Antibiotika, Antidepressiva, Antidiabetika, Antidiuretika, Anticholinergika, Antiarrhytmika, Antiemetika, Antitussiva, Antiepileptika, Antihistaminika, Antimykotika, Antisympathotonika, Antithrombotika, Androgenen, Antiandrogenen, Estrogenen, Antiestrogenen, Antiosteoporosemitteln, Antitumormitteln, Vasodilatatoren, anderen blutdrucksenkenden Mitteln, fiebersenkenden Mitteln, Schmerzmitteln, entzündungshemmenden Mitteln, β-Blockern, Immunsuppressiva und Vitaminen ausgewählt.

Einige nicht-beschränkende Beispiele von Arzneiwirkstoffen mit einer NH₂-Gruppe als Reaktionspartner der Kopplung mit HAS sind:
Albuterol, Alendronat, Amikazin, Ampicillin, Amoxicillin, Amphotericin B, Atenolol, Azathioprin, Cefaclor, Cefadroxil, Cefotaxim, Ceftazidim, Ceftriaxon, Cilastatin, Cimetidin, Ciprofloxacin, Clonidin, Colistin, Cosyntropin, Cycloserin, Daunorubicin, Doxorubicin, Desmopressin, Dihydroergotamin, Dobutamin, Dopamin, Ephedrin, Epinephrin, ε-Aminocapronsäure, Ergometrin, Esmolol, Famotidin, Flecainid, Folsäure, Flucytosin, Furosemid, Ganciclovir, Gentamicin, Glucagon, Hydrazalin, Imipenem, Isoproterenol, Ketamin, Liothyronin, Lutiliberin, Merpatricin, Metaraminol, Methyldopa, Metoclopramid, Metoprolol, Mexiletin, Mitomycin, Neomicin, Netilmicin, Nimodipin, Nystatin, Octreotid, Oxytocin, Pamidronat, Pentamidin, Phentolamin, Phenylephrin, Procainamid, Procain, Propranolol, Ritodrin, Sotalol, Teicoplanin, Terbutalin, Thiamin, Tiludronat, Tolazolin, Trimethoprim, Tromethamin, Vancomycin, Vasopressin und Vinblastin.

Bevorzugte Beispiele von Arzneiwirkstoffen mit einer NH₂-Gruppe als Reaktionspartner der Kopplung mit HAS sind 6-Aminopenicillinsäure, 7-Aminocephalosporin, 7-Aminocephalosporansäure und 7-Aminopenicillansäure.

Spezielle Beispiele für solche Wirkstoffe mit einer COOH-Gruppe als Reaktionspartner für die Kopplung mit HAS sind:
Acetylcystein, Azlocillin, Aztreonam, Benzylpenicillin, Camptothecin, Cefamandol, Cefazolin, Cefepim, Cefotaxim, Cefotetan, Cefoxitin, Ceftazidim, Ceftriaxon, Cephalothin, Cilastatin, Ciprofloxacin, Clavulansäure, Dicloxacillin, ε-Aminocapronsäure, Floxacillin, Folinsäure, Furosemid, Fusidinsäure, Imipemem, Indomethacin, Ketorolac, Liothyronin, Melphalan, Methyldopa, Piperacillin, Prostacyclin, Prostaglandine, Teicoplanin, Ticarcillin und Vancomycin.

Spezielle Beispiele für solche Wirkstoffe mit einer Aryl-OH-Gruppe als Reaktionspartner der Kopplung mit HAS sind:
Albuterol, Allopurinol, Apomorphin, Ceftriaxon, Dobutamin, Dopamin, Doxycyclin, Edrophonium, Isoproterenol, Liothyronin, Metaraminol, Methyldopa, Minocyclin, Pentazocin, Phenylephrin, Phentolamin, Propofol, Rifamycine, Ritodrin, Teicoplanin, Terbutalin, Tetracyclin und Vancomycin.

Spezielle Beispiele für solche Wirkstoffe mit einer aliphatischen OH-Gruppe als Reaktionspartner der Kopplung mit HAS sind Taxol und Palcitaxel.

Die Reaktionsprodukte der oben beschriebenen chemischen Kopplung können mit bekannten Verfahren untersucht und die Kopplungseffizienz festgestellt werden. Beispielsweise kann eine UV-Eichkurve für die betreffende niedermolekulare Substanz erstellt und dazu verwendet werden, den Gehalt an niedermolekularer Substanz in der Probe bzw. den Anteil der niedermolekularen Substanz am Kopplungsprodukt zu bestimmen. Falls die niedermolekulare Substanz keine UV-Absorption zeigt, können entsprechende kolorimetrische oder elektrochemische Nachweisverfahren in Analogie zu bekannten Verfahren entwickelt werden. Der Saccharidanteil im Konjugat ist beispielsweise durch eine Glykan-spezifische Färbung der aufgetrennten Reaktionsprodukte nachweisbar. Auch eine quantitative Glykanbestimmung ist möglich. Die Kopplungsausbeute von Reaktionen unter Beteiligung primärer Amine könnte auch durch Derivatisierung der nicht-umgesetzten Amine mit Fluorescamin und Bestimmung der Fluoreszenz festgestellt werden.

Die verbesserte Wasserlöslichkeit kann bei schwerlöslichen Ausgangsstoffen einfach durch Lösungsversuche überprüft werden. Im Falle der Kopplung mit teilweise wasserlöslichen Arzneiwirkstoffen kann die erhöhte Hydrophilie mittels einer OECD-Methode zur Ermittlung des IogP-Wertes bestimmt werden. Diese korreliert die Retentionszeit von Substanzen in der RP-HPLC mit dem Verteilungskoeffizienten in einem n-Octanol/Wasser-Gemisch. Alle mit dieser Methode untersuchten erfindungsgemäßen HES-Korijugate eluierten im Ausschlußvolumen einer C18-Säule, zeigten also keine Wechselwirkungen mit dem C18-Material.

Die Konjugate der vorliegenden Erfindung können gegebenenfalls als solche oder in Form einer pharmazeutischen Zusammensetzung zur prophylaktischen oder therapeutischen Behandlung des menschlichen oder tierischen Körpers eingesetzt werden.

Derartige Zusammensetzungen umfassen eine pharmazeutisch wirksame Menge eines erfindungsgemäßes Konjugats als aktiven Bestandteil sowie einen pharmazeutisch geeigneten Träger und gegebenenfalls andere therapeutische bzw. galenische Bestandteile oder Hilfsstoffe. Hilfsstoffe können z. B. Verdünnungsmittel, Puffer, Geschmacksmittel, Bindemittel, oberflächenaktive Mittel, Verdickungsmittel, Gleitmittel, Konservierungsstoffe (einschließlich Antioxidantien) sowie Substanzen einschließen, die dazu dienen, die Formulierung mit dem Blut des vorgesehenen Empfängers isotonisch zu machen. Eine pharmazeutisch wirksame Menge ist diejenige Menge, welche ausreicht, um bei einmaliger oder mehrmaliger Verabreichung im Rahmen einer Behandlung zur Erleichterung, Heilung oder Verhütung eines Krankheitszustands eine gewünschte positive Wirkung zu entfalten. Ein pharmazeutisch annehmbarer Träger ist ein Träger, der sowohl mit dem Arzneiwirkstoff als auch mit dem Körper des Patienten kompatibel ist.

Die Form der Zusammensetzung wird je nach dem gewünschten bzw. geeigneten Verabreichungsweg variieren. Geeignete Verabreichungswege können beispielsweise die orale, parenterale, z. B. subkutane, intramuskuläre,intravenöse, intraarterielle, intraartikuläre, intrathekale, extradurale Injektion bzw. gegebenenfalls Infusion, intranasale, intratracheale, rektale oder topische Verabreichung sein. Die pharmazeutischen Zusammensetzungen können günstigerweise in Form einer Dosierungseinheit dargeboten werden und nach irgendeinem der auf dem Gebiet der Pharmazie wohlbekannten Verfahren hergestellt werden.

Die HAS-Konjugate der vorliegenden Erfindung können auch auf allen anderen Gebieten eingesetzt werden, bei denen andere Polymer-Konjugate, z. B. PEG-Konjugate, zur Anwendung kamen. Einige spezielle, nicht beschränkende Beispiele sind die Verwendung eines HAS-Konjugats als immobilisierter Reaktionspartner für eine Reaktion in heterogener Phase oder als Säulenmaterial für die Affinitätschromatographie. Weitere Anwendungsmöglichkeiten werden für den Fachmann in Kenntnis der hier offenbarten Eigenschaften der erfindungsgemäßen HAS-Konjugate unschwer ersichtlich sein.

Die folgenden Beispiele sollen die Erfindung näher erläutern, ohne diese jedoch darauf zu beschränken. Insbesondere können analoge Reaktionen auch mit Hydroxymethylstärke und Hydroxypropylstärke durchgeführt und ähnliche Ergebnisse erzielt werden.

### BEISPIEL 1

### Selektive Oxidation von Hydroxyethylstärke (HES) mit lod

In einem Rundkolben wurden 10 g HES-130 kD in 12 ml entionisiertem Wasser unter Erwärmen gelöst. Zu dieser Lösung wurden 2 ml einer I₂-Lösung (0.1 N) gegeben. Eine Pipette mit 2 ml 1.0 N NaOH wurde mit dem Kolben über ein 2-Wege-Verbindungsstück verbunden und die NaOH-Lösung mit ca. 1 Tropfen pro 4 Minuten zugetropft. Nach Zugabe von ungefähr 0.2 ml der NaOH-Lösung war die Lösung entfärbt, zu diesem Zeitpunkt wurde eine zweite Portion von 2 ml 0.1 N lodlösung zugegeben. Die Reaktion war nach Zugabe von insgesamt 14 ml lodlösung und 2.8 ml NaOH-Lösung beendet. Die Reaktionsmischung wurde anschließend gegen entionisiertes Wasser dialysiert.

### Lactonisierung:

Die teilweise entsalzte Lösung wurde einer Chromatographie an einer Kationenaustauschersäule (Amberlite IR-120, H⁺-Form) unterzogen, um die Aldonatgruppen in Aldonsäuregruppen umzuwandeln. Anschließend wurde das Wasser durch Lyophilisation entfernt und so die Lactonform erhalten.

### Bestimmung des Oxidationsgrades:

Zu jeweils 1 ml Probenlösung werden unter N₂-Atmosphäre 1 ml alkalisches Kupferreagenz (3.5 g Na₂PO₄ 4.0 g K-Na-Tatrat in 50 ml H₂O, dazu 10 ml 1 N NaOH, 8.0 ml 10%iger (Gew./Vol.) CuSO₄-Lösung und 0.089 g K-lodat in 10 ml H₂O, nach Zugabe von 18 g Na-Sulfat auf 100 ml auffüllen) pipettiert. Es wird 45 Minuten bei 100 °C erhitzt. Nach Abkühlen werden 0.2 ml 2.5%iger KI-Lösung und 0.15 ml 2 M H₂SO₄ addiert. Nach 5 Min. wird mit 1 Tropfen Phenolrot-Indikatorlösung (1 % Gew./Vol.) versetzt und mit 5 mM Na₂S₂O₃-Lösung bis zum Verschwinden der Farbe titriert. Aus dem Verbrauch an Titrationsmittel lässt sich die Konzentration nichtumgesetzter Aldehydgruppen berechnen.

Es wurde eine annähernd quantitative Ausbeute erzielt (>98%). Hydroxyethylstärken mit höherer molarer Masse (z. B. 130 kD, 250 kD, 400 kD) konnten, genauso wie Hydroxyethylstärken mit niedrigerer molarer Masse (z. B. 10 kD, 25 kD, 40 kD), nach dieser Vorschrift in ähnlich hohen Ausbeuten oxidiert werden.

### BEISPIEL 2

### Selektive Oxidation von HES mit Cu²⁺-lonen

Unter Erwärmen wurde eine Lösung von 0.24 mMol HES-130 kD in 10 ml entionisiertem Wasser hergestellt. Diese Lösung wurde in einem 100-ml-Rundkolben auf eine Temperatur von 70-80 °C erhitzt und mit 1,17 mMol stabilisiertem Cu²⁺ (z. B. Rochelle-Salz als Stabilisator oder andere Stabilisatoren) und verd. wässriger NaOH-Lösung versetzt (Endkonzentration 0,1 N NaOH). Danach wurde die Temperatur auf 100 °C erhöht und die Reaktion solange laufen lassen, bis eine rötliche Farbe entstanden war. Die Reaktion wurde gestoppt und die Reaktionsmischung auf 4 °C abgekühlt. Der rötliche Niederschlag wurde durch Filtration entfernt. Das Filtrat wurde gegen entionisiertes Wasser dialysiert und anschließend wie in Beispiel 1 in das Lacton umgewandelt. Die Oxidation verlief quantitativ (Ausbeute >99%). Nach dieser Methode ließen sich auch niedermolekulare HES (z. B. HES-10 kD, HES-25 kD, HES-40 kD) und höhermolekulare HES-Spezies (z. B. 130 kD, 250 kD, 400 kD) oxidieren.

### BEISPIEL 3

### Kopplung von selektiv oxidierter Hydroxyethylstärke (ox-HES) an Alendronat

In einem 100-ml-Rundkolben wurden 5 mg Alendronat (ein Bisphosphonat) und ein 3-5facher molarer Überschuß an ox-HES Lacton (hergestellt wie in Beispiel 1 oder 2 beschrieben) in 4-5 ml DMSO gelöst. Die Suspension wurde auf 70°C erwärmt und unter mäßigem Rühren (Magnetrührer) 24-36 Stunden belassen. Die Reaktion wurde dann abgebrochen und die Reaktionsmischung auf Raumtemperatur abgekühlt. Danach wurden 20-30 ml Wasser zugegeben und diese Lösung gegen destilliertes Wasser dialysiert. Anstelle der Dialyse kann auch eine Ultrafiltration mit einer geeigneten Ausschlussgrenze der Membran eingesetzt werden. Dies ermöglicht nicht nur den Austausch des Lösungsmittels sondern auch eine Konzentration der Lösung, welche anschließend lyophilisiert wird. Der Erfolg der Kopplung wird mittels analytischer Standardverfahren, z. B. Gelpermeationschromatographie und Ninhydrin-Test auf freie Aminogruppen, nachgewiesen. Die Ausbeute an Kopplungsprodukt betrug ca. 85% für die Kopplung mit ox-HES-130 kD und ca. 80 % für die Kopplung mit ox-HES-10 kD Lacton.

### BEISPIEL 4

### Kopplung von selektiv oxidierter HES (ox-HES) an Amphotericin B

12.0 g getrocknetes ox-HES-130 kD Lacton wurden in 30 ml trockenem DMSO in einer N₂-Atmosphäre gelöst. Die Lösung wurde auf 70 °C erwärmt und 52 mg Amphotericin B zugegeben. Unter Lichtausschluss wurde die Reaktion für 24 h unter diesen Bedingungen belassen. Der Nachweis der erfolgreichen Kopplung wurde durch Gelpermeationschromatographie mit photometrischer Detektion bei 385 nm (λₘₐₓ des Amphotericins) geführt. Nach Abschluss der Reaktion wurde durch Zugabe von 80 ml destilliertem Wasser gestoppt und intensiv gegen Wasser dialysiert. Lyophilisation ergab ein schwach gelbes Kopplungsprodukt. (Ausbeute ca. 87%).

Unter vergleichbaren Bedingungen wurde bei der Kopplung von ox-HES-10 kD Lacton mit Amphotericin B eine Ausbeute von ca. 75% erzielt.

### BEISPIEL 5

### Kopplung von ox-HES an Ampicillin

In einem 100-ml-Rundkolben wurden 1.3 g trockenes ox-HES-130 kD Lacton in 5 ml trockenem DMSO gelöst. Diese Lösung wurde auf 45°C erwärmt und 11.0 mg Ampicillin (Aldrich # 27.186-1) zugegeben. Die Reaktion lief unter moderatem Rühren für 20 h und wurde nach dieser Zeit durch die Zugabe von 25 ml destilliertem Wasser abgestoppt. Die Reaktionsmischung wurde gegen destilliertes Wasser dialysiert und anschließend lyophilisiert. Der Kopplungserfolg wurde durch Analyse des Produkts mit GPC und Bestimmung der freien Aminogruppen am Ampicillin mit Ninhydrin nachgewiesen.

### BEISPIEL 6

### Kopplung von ox-HES an Neomycin

3 x 10⁻⁵ Mol ox-HES-25 kD Lacton wurden in 5 ml N-Methylpyrrolidon bei 60°C in einem 50-ml-Reaktionsgefäß unter Magnetrühren gelöst. Nach der Zugabe von 10 mg Neomycin in 2 ml trockenem DMSO wurde für ca. 10 h zum Rückfluss gekocht. Nach Abkühlung auf Raumtemperatur beendete die Zugabe von weiteren 35 ml Wasser die Reaktion. Durch Dialyse wurde der größte Teil der Solventien entfernt und das Kopplungsprodukt anschließend lyophilisiert. Durch GPC mit UV-Detektion konnte Kopplungsprodukt mit ca. 82 % Ausbeute nachgewiesen werden.

### BEISPIEL 7

### Kopplung von ox-HES an Mepartricin

10 ml Ethylenglcol waren notwendig, um 2.5 g ox-HES-130 kD Lacton und 22 mg Mepartricin (erhältlich von Societä Prodotti Antibiotici, Mailand, Italien) unter Erwärmen vollständig zu lösen. Das Lösungsmittel war zuvor entgast und getrocknet worden. Die Reaktionslösung wurde 36 h lang unter Lichtausschluss und einer Inertgas-Atmosphäre gerührt und die Reaktion schließlich durch den Eintrag von 40 ml eiskaltem Wasser gestoppt. Das Ethylenglycol wurde mittels Ultrafiltration (10-kD-Membran) abgetrennt, anschließende Lyophilisierung ergab 2.1 g schwach gelbliches Pulver. Die weitere Reinigung erfolgte durch RP-HPLC an einer C18-Säule mit UV/VIS-Detektion.

### BEISPIEL 8

### Kopplung von ox-HES an Nystatin

In einem 100-ml-Rundkolben wurden 2.5 g trockenes ox-HES-130 kD Lacton in 10 ml trockenem DMSO gelöst. Nach Zugabe von 9.5 mg Nystatin wurde auf 60 °C erwärmt und unter Inertgas-Atmosphäre und im Dunkeln gerührt. Die Reaktion lief unter moderatem Rühren für 48 h und wurde nach dieser Zeit durch Zugabe von 50 ml destillierten Wasser abgestoppt. Die Reaktionsmischung wurde gegen dest. Wasser dialysiert und anschließend lyophilisiert. Die erfolgreiche Kopplung konnte durch RP-HPLC (C18-Säule) und Detektion bei 325 nm nachgewiesen werden. Die Ausbeute, anhand der Absorption des Produktpeaks abgeschätzt, betrug ca. 67%.

### BEISPIEL 9

### Kopplung von ox-HES an Mitomycin C

2.5 g ox-HES-130 kD Lacton und 20 mg Mitomycin (Fluka # 69824) wurden in 10 ml einer 9:1-Mischung von DMSO:MeOH bei 60°C gelöst. Die Reaktionslösung wurde 24 h unter Rückfluss gehalten und danach wurden zum Abstoppen der Reaktion 40 ml Wasser zugegeben. Diese Lösung wurde über Nacht gegen entionisiertes Wasser dialysiert und anschließend einer Gefriertrocknung unterzogen. Der Nachweis der Kopplung wurde mittels RP-HPLC und Detektion bei 320 nm geführt. Das erwartete Kopplungsprodukt entstand in einer Ausbeute von 82%.

### BEISPIEL 10

### Kopplung von ox-HES an Daunorubicin

1.3 g ox-HES-130 kD Lacton wurden in 10 ml N-Methylpyrrolidon bei 70 °C unter Rühren gelöst. Dazu wurden 17 mg Daunorubicin (Fluka #30450), gelöst in 3 ml DMF, tropfenweise zugegeben. Die Reaktionsmischung wurde 20 h lang unter diesen Bedingungen gerührt, auf Raumtemperatur abgekühlt und schließlich mit 40 ml destilliertem Wasser geschüttelt. Mittels Dialyse gegen Wasser wurde der Hauptteil der Lösungsmittel entfernt und anschließend gefriergetrocknet. Das gekoppelte Daunorubicin wurde durch RP-HPLC und UV-VIS-Detektion nachgewiesen.

### BEISPIEL 11

### Kopplung von ox-HES an 7-Aminocephalosporin

In einem 100-ml-Rundkolben wurden 3.0 g ox-HES-130 kD Lacton und 20 mg 7-Aminocephalosporin (Fluka #07300) in 5 ml trockenem DMSO unter magnetischem Rühren gelöst. Die Temperatur wurde auf 50°C erhöht und für 15 h gehalten. Nach dieser Zeit wurde auf 25 °C abgekühlt und die Reaktionsmischung durch Zugabe von 5 ml destilliertem Wasser verdünnt. DMSO und nicht umgesetztes 7-Aminocephalosporin wurden mittels Dialyse gegen destilliertes Wasser entfernt. Die Lösung wurde anschließend lyophilisiert und das Produkt durch DC und GPC analysiert.

### BEISPIEL 12

### Kopplung von ox-HES an 6-Aminopenicillinsäure

Die in Beispiel 11 beschriebene Reaktion wurde auch mit 16 mg 6-Aminopenicillinsäure an Stelle des 7-Aminocephalosporins unter denselben Bedingungen durchgeführt und das Reaktionsprodukt unter denselben Bedingungen aufgearbeitet und analysiert.

### BEISPIEL 13

### Kopplung von ox-HES an LHRH

1,0 g getrocknetes ox-HES-130 kD Lacton wurde mit 5 mg LHRH (Lutiliberin, "luteinizing hormone releasing hormone") (Bachem, Schweiz) in 10 ml trockenem DMSO inkubiert. Die Reaktion ging 15 h lang bei 45°C unter Rühren vonstatten und wurde durch die Zugabe von 40 ml Wasser gestoppt. Hesyliertes LHRH wurde durch Lyophilisierung gewonnen, nachdem es extensiv gegen Wasser dialysiert worden war, um die Hauptmenge an DMSO und nicht umgesetztes Peptid zu entfernen. Das entstandene Produkt wurde mit GPC (Superose 12, Amersham-Pharmacia, Schweden) und UV-Detektion bei 280 nm analysiert. Eine Stöchiometrie von ungefähr 1:1 für das Kopplungsprodukt ergab sich aus der Quantifizierung des Peptids auf der Basis der Trp-Absorption und der Quantifizierung des Polysaccharid-Anteils durch die Phenol/Schwefelsäure-Färbung.

### Beispiel 14

### Kopplung von ox-HES an Camptothecin

In einem Rundkolben wurden 20 mg Camptothecin in 5 ml trockenem DMSO bei 50 °C gelöst. Zu dieser Lösung wurden 36 mg 1.4-Diaminobutan in 2 ml trockenem DMSO getropft. Die Reaktionsmischung wurde für 24 h unter diesen Bedingungen und leichtem Rühren belassen. Das Konjugationsprodukt wurde durch Flashchromatographie gereinigt. Die Ausbeute betrug ca. 83%.

Für die Kopplungsreaktion des modifizierten Camptothecins mit ox-HES-130 kD wurde der gesamte Reaktionsansatz nach Reinigung zusammen mit 3.6 g des Polysaccharid-Lactons in 8 ml trockenem DMSO unter Rühren und Erwärmen auf 50 °C gelöst. Der Verlauf der Reaktion wurde durch RP-HPLC von Proben aus der Reaktionsmischung verfolgt. Nach 20 h bei 50 °C konnte keine weitere Produktbildung mehr beobachtet werden und die Reaktion wurde durch Zugabe von 50 ml destilliertem Wasser gestoppt. Nach Dialyse gegen Wasser wurde das Kopplungsprodukt gefriergetrocknet. Die Analyse erfolgte durch GPC und Anfärbung der freien Aminogruppe im modifiziertem, nicht-umgesetztem Camptothecin mit Ninhydrin auf einer DC-Platte.

### BEISPIEL 15

### Kopplung von ox-HES an Prostacyclin

### a) Aminofunktionalisierung

352 mg Prostacyclin (Sigma-Aldrich) wurden in 5 ml trockenem DMF mit 2% Methylenchlorid (V/V) bei 0°C gelöst. Dazu wurden 1.3 g Dicyclohexylcarbodiimid (DCC) in 5 ml trockenem DMF gegeben. Unter leichten Rühren ließ man 30 Minuten reagieren. Anschließend wurde ein 5-fach molarer Überschuss (bezogen auf Prostacyclin) an 1.5-Diaminoethylether addiert und die Lösung langsam auf Raumtemperatur erwärmt. Die Reinigung des aminofunktionalisierten Kopplungsproduktes erfolgte mit Flashchromatographie an Silica-Phase.

### b) Hesylierung

220 mg des gereinigten Kopplungsproduktes aus a) wurden in 8 ml Glycol bei Raumtemperatur gelöst. Dazu wurden 4.0 g ox-HES-130 kD Lacton, gelöst in 10 ml Glycol, unter Rühren gemischt und auf 45 °C erwärmt. Nach einer Reaktionszeit von 8 h wurde im Eisbad abgekühlt und intensiv gegen Wasser dialysiert. Die klare Lösung wurde mit RP-HPLC an einer C18-Säule untersucht. Aus dem Verhältnis der Flächen im Ausschlussvolumen der Säule (Kopplungsprodukt) und der Ausgangssubstanz konnte die Kopplungseffizienz berechnet werden. Die Ausbeute betrug 53%.

### BEISPIEL 16

### Kopplung von HES an Alendronat

In einem 100-ml-Rundkolben wurde ein zehnfacher molarer Überschuss an HES-25 kD zu einer Lösung von 2.25 mg Alendronat in 4 ml Phosphatpuffer (0.1 M, pH 7.5) zugegeben. Die Reaktionsmischung wurde geschüttelt, um das Polysaccharid vollständig zu lösen, und dann wurde ein dreißigfacher molarer Überschuss an NaBH₃CN zugegeben. Die Reaktion lief bei Raumtemperatur 48 h lang ab, wobei die Entstehung eines Kopplungsprodukts an einem Aliquot durch Umsetzung mit Fluorescamin, das mit freien Aminogruppen ein fluoreszierendes Produkt ergibt, nachgewiesen wurde.

### BEISPIEL 17

### Kopplung von HES an Amoxillin

In einem Zweihalskolben wurden 4.0 ml 0.1 N Na-Phosphatpuffer (pH 7.5) vorgelegt und darin 1.5 g HES-40 kD durch Erwärmen auf 60 °C gelöst. Nach Abkühlen auf 25 °C wurden 7.0 mg Amoxillin (Fluka #10039) unter magnetischem Rühren zugegeben. In einem separaten Gefäß wurde eine Lösung von NaBH₃CN, die einem dreißigfachen molaren Überschuss entsprach, in 2 ml des gleichen Na-Phosphatpuffers hergestellt. Mit Hilfe eines Tropftrichters wurde die Cyanoborhydrid-Lösung langsam der ersten Lösung in einem Zeitraum von 30 Minuten zugetropft. Die Reaktionsmischung wurde 24-36 h lang weitergerührt und dann der pH-Wert zum Stoppen der Rektion mit 0.1 N HCl auf 4 eingestellt. Die Lösung wurde durch Dialyse entsalzt und lyophilisiert. Der Nachweis des Kopplungsprodukt erfolgte mittels GPC und UV-Photometer.

### BEISPIEL 18

### Kopplung von HES an Cefaclor

In einem 100-ml-Rundkolben wurden 4 ml 0.1 N Na-Phosphatpuffer (pH 7.0) dazu verwendet, 110 mg NaBH₃CN zu lösen. Unter Rühren wurden 6.0 x 10⁻⁵ Mol HES-130 kD und 2.0 x 10⁻⁵ Mol Cefaclor (Fluka #22125) zugegeben. Die Reaktionstemperatur wurde bei 25 °C gehalten und die Reaktionsmischung 24 h lang moderat gerührt. Danach wurde die Lösung auf pH 4.0 angesäuert und weitere 30 Minuten lang gerührt. Zur Entsalzung und Konzentration wurde ultrafiltriert (10-kD-Membran). Das Kopplungsprodukt wurde mittels HP-GPC bei 265 nm nachgewiesen.

### BEISPIEL 19

### Kopplung von HES an Doxorubicin

6.0 mg Doxorubicin (Fluka #45584) wurden in Gegenwart eines dreifachen molaren Überschusses an HES-130 kD in 4 ml 0.1 N Na-Phosphatpuffer (pH 7.5) bei Raumtemperatur suspendiert. Die Reaktionsmischung wurde 30 Minuten lang stark gerührt und langsam mit 3 ml einer 0.8 M NaBH₃CN-Lösung versetzt. Die Reaktion wurde für 48 h lang bei Raumtemperatur unter Rühren gehalten. Anschließend wurde mit einer 10-kD-Membran diafiltriert, um Salze und nicht umgesetztes Doxorubicin zu entfernen. Die diafiltrierte Lösung wurde lyophilisiert und das Kopplungsprodukt mit GPC und RP-HPLC untersucht.

### BEISPIEL 20

### Kopplung von HES an Vasopressin

In einem Rundkolben, der mit einem Tropftrichter versehen war, wurden 1.25 g HES-130 kD in 5 ml 0.1 M Na-Phosphatpuffer, pH 8.0, unter Erwärmen und leichtem Rühren gelöst. Zu dieser Lösung wurden 5 mg Vasopressin (Bachem, Schweiz) zugegeben. 30 mg NaBH₃CN wurden in 2 ml 0.1 M Phosphatpuffer (pH 7.5) gelöst und langsam über den Tropftrichter der Reaktionsmischung zugetropft. Die Reaktion wurde etwa 24 h lang bei 25 °C stehen gelassen. Zur Beendigung der Reaktion wurde der pH-Wert durch die Zugabe von 0.1 N HCl auf 4,0 abgesenkt. Nach extensiver Dialyse gegen Wasser wurde das hesylierte Produkt gefriergetrocknet. Die Analyse erfolgte mit GPC wie oben beschrieben und UV-Detektion bei 220 nm.

### BEISPIEL 21

### Kopplung von ox-HES 70 kD an Neomycin

In einem Zweihalskolben wurden unter Argonatmosphäre 1.01 mg Neomycin (Sulfat-Salz) und 126.21 mg oxHES 70 kD in 2 ml DMSO aufgelöst und nach Zugabe von 0.81 mg DMAP 24 h bei 70 °C erhitzt. Anschließend wurde die Reaktion durch Zugabe von Aceton beendet, wobei das Kopplungsprodukt ausfiel. Der Feststoff wurde in Wasser aufgelöst und 48 h durch Dialyse gegen Wasser aufgereinigt. Nach Gefriertrocknung erhielt man 80 mg weißes Kopplungsprodukt (63 %).

### BEISPIEL 22

### Alternative Methode der Kopplung von ox-HES 70 kD an Neomycin

Die Kopplung von Neomycin an ox-HES 70 kD ließ sich ebenfalls erfolgreich bei Raumtemperatur in DMSO mit Zugabe von EDC als Aktivator durchführen. Hierfür wurden 16.97 mg Neomycin (Sulfat-Salz), 348 mg ox-HES 70 kD und 2.28 mg DMAP in 1 ml DMSO aufgelöst. Nach Zugabe von 3.83 mg DCC (1 Äquivalent) rührte man die Lösung 2 h und wiederholte die Zugabe eines Äquivalents DCC. Dieses Verfahren wurde wiederholt, bis 10 Äquivalente DCC in die Reaktionslösung zugegeben wurden. Die Reaktionszeit betrug insgesamt 24 h. Nach Zugabe von 20 ml Aceton in die Lösung fiel das Kopplungsprodukt aus. Der Feststoff wurde in Wasser aufgelöst und 48 h durch Dialyse gegen Wasser aufgereinigt. Nach Gefriertrocknung erhielt man 280 mg weißes Kopplungsprodukt (80 %).

### BEISPIEL 23

### Kopplung von ox-HES 70 kD an Daunorubicin

In einem Zweihalskolben wurden 0.5 mg Daunorubicin-Hydrochlorid, 829.2 mg ox-HES 70 kD und 0.108 mg DMAP in 2 ml DMSO unter Argonatmosphäre gelöst und 24 h bei 70 °C erhitzt. Anschließend gab man Aceton (20 ml) dazu, wobei das Kopplungsprodukt ausfiel. Die Lösung wurde zentrifugiert und der Präzipitat mehrmals mit Aceton gewaschen und zentrifugiert. Man erhielt einen leicht rosagefärbten Feststoff, der in Wasser aufgelöst und gegen Wasser dialysiert wurde. Nach Gefriertrocknung erhält man 656 mg (80 %) eines leicht rosa gefärbten Feststoffes. Die Reinheit des gekoppelten Daunorubicins wurde mittels RP-HPLC überprüft.

### BEISPIEL 24

### Kopplung von ox-HES 130 kD an 7-Aminocephalosporansäure

In einem 100-ml-Rundkolben wurden 383 mg ox-HES-130 kD und 1.22 mg 7-Aminocephalosporansäure (Fluka #07300) in 2 ml trockenem DMSO unter magnetischem Rühren gelöst. Die Temperatur wurde auf 70°C erhöht und für 24 h gehalten. Nach dieser Zeit wurde auf 25 °C abgekühlt und das Reaktionsprodukt durch Zugabe von 20 ml Aceton ausgefällt. Der Feststoff wurde mit 20 ml Aceton gewaschen und in 20 ml destilliertem Wasser aufgelöst. Die weitere Reinigung des Kopplungsproduktes erfolgte mittels Dialyse gegen destilliertes Wasser. Die Lösung wurde anschließend lyophilisiert und das Produkt durch DC und GPC analysiert. Man erhielt 270 mg Kopplungsprodukt (70 %) in Form eines weißen Feststoffes.

### BEISPIEL 25

### Kopplung von ox-HES 70 kD an 6-Aminopenicillansäure

Die in Beispiel d beschriebene Reaktion wurde auch mit 1.57 mg 6-Aminopenicillansäure an Stelle der 7-Aminocephalosporansäure und 135.54 mg ox-HES 70 kD unter denselben Bedingungen durchgeführt. Das Reaktionsprodukt wurde unter denselben Bedingungen aufgearbeitet und analysiert. Nach der Aufreinigung erhielt man 88 mg Kopplungsprodukt (65 %) als weißen Feststoff.

### BEISPIEL 26

### Kopplung von HES 40 kD an Amoxicillin

In einem Zweihalskolben wurden 4.0 ml 0.1 N Na-Phosphatpuffer (pH 7.5) vorgelegt und darin 1.5 g HES-40 kD durch Erwärmen auf 60 °C gelöst. Nach Abkühlen auf 25 °C wurden 7.0 mg Amoxicillin (Fluka #10039) unter magnetischem Rühren zugegeben. In einem separaten Gefäß wurde eine Lösung von NaBH₃CN, die einem dreißigfachen molaren Überschuss entsprach, in 2 ml des gleichen Na-Phosphatpuffers hergestellt. Mit Hilfe eines Tropftrichters wurde die Cyanoborhydrid-Lösung langsam der ersten Lösung in einem Zeitraum von 30 Minuten zugetropft. Die Reaktionsmischung wurde 24-36 h lang weitergerührt und dann der pH-Wert zum Stoppen der Rektion mit 0.1 N HCl auf 4 eingestellt. Die Lösung wurde durch Dialyse entsalzt und lyophilisiert. Der Nachweis des Kopplungsprodukt erfolgte mittels GPC und UV-Photometer.

### BEISPIEL 27

### Kopplung von ox-HES 70 kD an Amoxicillin

In einem 100-ml-Rundkolben wurden 173 mg ox-HES 70 kD und 0.85 mg Amoxicillin in 2 ml trockenem DMSO unter magnetischem Rühren gelöst. Die Temperatur wurde auf 70°C erhöht und für 24 h gehalten. Nach dieser Zeit wurde auf 25 °C abgekühlt und das Reaktionsprodukt durch Zugabe von 20 ml Aceton ausgefällt. Der Feststoff wurde mit 20 ml Aceton gewaschen und in 20 ml destilliertem Wasser aufgelöst. Die weitere Reinigung des Kopplungsproduktes erfolgte mittels Dialyse gegen destilliertes Wasser. Die Lösung wurde anschließend lyophilisiert und das Produkt durch DC und GPC analysiert. Man erhält 151 mg Kopplungsprodukt (87 %) in Form eines weißen Feststoffes.

### BEISPIEL 28

### Kopplung von ox-HES 70 kD an Cefadroxil

In einem 100-ml-Rundkolben wurden 610 mg ox-HES 70 kD und 2.965 mg Cefadroxil in 2 ml trockenem DMSO unter magnetischem Rühren aufgelöst. Die Temperatur wurde auf 70°C erhöht und für 24 h gehalten. Anschließend kühlte man die Temperatur auf 25 °C ab und fällte das Reaktionsprodukt durch Zugabe von 20 ml Aceton aus. Der Feststoff wurde mit 20 ml Aceton gewaschen und in 20 ml destilliertem Wasser aufgelöst. Die weitere Reinigung des Kopplungsproduktes erfolgte mittels Dialyse gegen destilliertes Wasser. Die Lösung wurde anschließend lyophilisiert und das Produkt durch DC und GPC analysiert. Man erhielt 490 mg Kopplungsprodukt (87 %) in Form eines weißen Feststoffes.

### BEISPIEL 29

### Kopplung von ox-HES 70 kD an Glucagon

In einem Rundkolben werden Glucagon (66 x 10⁻⁹ mol, 0.23 mg), oxHES 70 kD (6.6 x 10⁻⁶ mol, 123 mg) in 1 ml DMSO gelöst. In Zeitabständen von 1 h gibt man in 8 Portionen DDC hinzu bis insgesamt 23.08 mg in die Reaktionslösung zugegeben worden sind. Nach einer Reaktionszeit von 24 h, wird die Reaktion durch Zugabe von 15 ml Wasser abgebrochen. Das Kopplungsprodukt durch Dialyse gegen Wasser aufgereinigt. Nach Gefriertrocknung erhält man 79 mg weißes Kopplungsprodukt (65 %).

Im Folgenden werden bevorzugte Ausführungsformen der vorliegenden Erfindung, d.h. bevorzugte Verfahren und Konjugate der Erfindung sowie deren Verwendung beschrieben, einschließlich bevorzugter Kombinationen diese Ausführungsformen:
1. Konjugat von Hydroxyalkylstärke und einer niedermolekularen Substanz, **dadurch gekennzeichnet, dass** die bindende Wechselwirkung zwischen dem Hydroxyalkylstärkemolekül und der niedermolekularen Substanz auf einer kovalenten Bindung beruht, welche das Ergebnis einer Kopplungsreaktion zwischen (i) der endständigen Aldehydgruppe oder einer aus dieser Aldehydgruppe durch chemische Umsetzung hervorgegangenen funktionellen Gruppe des Hydroxyalkylstärkemoleküls und (ii) einer mit dieser Aldehydgruppe oder daraus hervorgegangenen funktionellen Gruppe des Hydroxyalkylstärkemoleküls reaktionsfähigen funktionellen Gruppe der niedermolekularen Substanz ist, wobei die bei der Kopplungsreaktion unmittelbar resultierende Bindung gegebenenfalls durch eine weitere Reaktion zur obengenannten kovalenten Bindung modifiziert sein kann.
2. Konjugat nach Ausführungsform 1, **dadurch gekennzeichnet, dass** die aus der endständigen Aldehydgruppe des Hydroxyalkylstärkemoleküls hervorgegangene funktionelle Gruppe eine der funktionellen Gruppen eines bifunktionellen Linkermoleküls ist, mit dem die endständige Aldehydgruppe oder eine daraus hervorgegangene funktionelle Gruppe umgesetzt wurde.
3. Konjugat nach Ausführungsform 1 oder 2, **dadurch gekennzeichnet, dass** die reaktionsfähige funktionelle Gruppe der niedermolekularen Substanz eine der funktionellen Gruppen eines bifunktionellen Linkermoleküls ist, welches an die niedermolekulare Substanz gekoppelt wurde.
4. Konjugat nach einer der Ausführungsformen 1 bis 3, **dadurch gekennzeichnet, dass** die kovalente Bindung das Ergebnis einer Kopplungsreaktion zwischen einer durch selektive Oxidation der endständigen Aldehydgruppe des Hydroxyalkylstärkemoleküls gebildeten Carboxylgruppe oder aktivierten Carboxylgruppe und einer primären Aminogruppe oder Thiolgruppe der niedermolekularen Substanz ist.
5. Konjugat nach Ausführungsform 4, **dadurch gekennzeichnet, dass** die kovalente Bindung eine Amidbindung ist, welche das Ergebnis einer Kopplungsreaktion zwischen einem durch selektive Oxidation der endständigen Aldehydgruppe des Hydroxyalkylstärkemoleküls gebildeten Lacton und einer primären Aminogruppe der niedermolekularen Substanz ist.
6. Konjugat nach einer der Ausführungsformen 1 bis 3, **dadurch gekennzeichnet, dass** die kovalente Bindung eine Aminbindung ist, welche das Ergebnis einer Kopplungsreaktion zwischen der endständigen Aldehydgruppe des Hydroxyalkylstärkemoleküls und einer primären Aminogruppe der niedermolekularen Substanz unter Bildung einer Schiffschen Base und der Reduktion der Schiffschen Base zum Amin ist.
7. Konjugat nach einer der Ausführungsformen 1 bis 6, **dadurch gekennzeichnet, dass** das Hydroxyalkylstärkemolekül ein Molekulargewicht im Bereich von etwa 70 bis etwa 1000 kD aufweist.
8. Konjugat nach Ausführungsform 7, **dadurch gekennzeichnet, dass** das Hydroxyalkyl- stärkemolekül ein Molekulargewicht von etwa 130 kD aufweist.
9. Konjugat nach einer der Ausführungsformen 1 bis 8, **dadurch gekennzeichnet, dass** das Hydroxyalkylstärkemolekül einen Substitutionsgrad von etwa 0,3 bis etwa 0,7 aufweist.
10. Konjugat nach einer der Ausführungsformen 1 bis 9, **dadurch gekennzeichnet, dass** das Hydroxyalkylstärkemolekül ein Verhältnis der C2-zu C6-Substitution von 8 bis 12 aufweist.
11. Konjugat nach einer der Ausführungsformen 1 bis 10, **dadurch gekennzeichnet, dass** das Hydroxyalkylstärkemolekül ein Hydroxyethylstärkemolekül ist.
12. Konjugat nach einer der Ausführungsformen 1 bis 11, **dadurch gekennzeichnet, dass** die niedermolekulare Substanz ein Arzneiwirkstoff ist.
13. Konjugat nach Ausführungsform 12, **dadurch gekennzeichnet, dass** der Arzneiwirkstoff aus der Gruppe aus Antibiotika, Antidepressiva, Antidiabetika, Antidiuretika, Anticholinergika, Antiarrhytmika, Antiemetika, Antiepileptika, Antihistaminika, Antimykotika, Antisympathotonika, Antithrombotika, Androgenen, Antiandrogenen, Estrogenen, Antiestrogenen, Antiosteoporosemitteln, Antitumormitteln, Vasodilatatoren und anderen blutdrucksenkenden Mitteln, fiebersenkenden Mitteln, Schmerzmitteln, entzündungshemmenden Mitteln, beta-Blockern, Immunsuppressiva und Vitaminen ausgewählt ist.
14. Konjugat nach Ausführungsform 12 oder 13, **dadurch gekennzeichnet, dass** die an der Kopplungsreaktion beteiligte funktionelle Gruppe des Arzneiwirkstoffs eine Aminogruppe ist.
15. Konjugat nach Ausführungsform 14, **dadurch gekennzeichnet, dass** der Arzneiwirkstoff aus der Gruppe aus Albuterol, Alendronat, Amikazin, Aminopenicillin, Amoxicillin, Atenolol, Azathioprin, Cefaclor, Cefadroxil, Cefotaxim, Ceftazidim, Ceftriaxon, Cilastatin, Cimetidin, Ciprofloxacin, Clonidin, Colistin, Cosyntropin, Cycloserin, Daunorubicin, Doxorubicin, Desmopressin, Dihydroergotamin, Dobutamin, Dopamin, Ephedrin, Epinephrin, epsilon-Aminocapronsäure, Ergometrin, Esmolol, Famotidin, Flecainid, Folsäure, Flucytosin, Furosemid, Ganciclovir, Gentamicin, Glucagon, Hydrazalin, Imipenem, Isoproterenol, Ketamin, Liothyronin, Lutiliberin, Merpatricin, Metaraminol, Methyldopa, Metoclopramid, Metoprolol, Mexiletin, Mitomycin, Neomycin, Netilmicin, Nimodipin, Nystatin, Octreotid, Oxytocin, Pamidronat, Pentamidin, Phentolamin, Phenylephrin, Procainamid, Procain, Propranolol, Ritodrin, Sotalol, Teicoplanin, Terbutalin, Thiamin, Tiludronat, Tolazolin, Trimethoprim, Tromethamin, Vancomycin, Vasopressin und Vinblastin ausgewählt ist.
16. Konjugat nach Ausführungsform 12 oder 13, **dadurch gekennzeichnet, dass** die an der Kopplungsreaktion beteiligte funktionelle Gruppe des Arzneiwirkstoffs eine Carboxylgruppe oder aktivierte Carboxylgruppe ist.
17. Konjugat nach Ausführungsform 16, **dadurch gekennzeichnet, dass** der Arzneiwirkstoff aus der Gruppe aus Acetylcystein, Azlocillin, Aztreonam, Benzylpenicillin, Camptothecin, Cefamandol, Cefazolin, Cefepim, Cefotaxim, Cefotetan, Cefoxitin, Ceftazidim, Ceftriaxon, Cephalothin, Cilastatin, Ciprofloxacin, Clavulansäure, Dicloxacillin, epsilon-Aminocapronsäure, Floxacillin, Folinsäure, Furosemid, Fusidinsäure, Imipemem, Indomethacin, Ketorolac, Liothyronin, Melphalan, Methyldopa, Piperacillin, Prostacyclin, Prostaglandine, Teicoplanin, Ticarcillin und Vancomycin ausgewählt ist.
18. Konjugat nach Ausführungsform 12 oder 13, **dadurch gekennzeichnet, dass** die an der Kopplungsreaktion beteiligte funktionelle Gruppe des Arzneiwirkstoffs eine aliphatische oder Aryl-OH-Gruppe ist.
19. Konjugat nach Ausführungsform 18, **dadurch gekennzeichnet, dass** der Arzneimittel- wirkstoff aus der Gruppe aus Albuterol, Allopurinol, Apomorphin, Ceftriaxon, Dobutamin, Dopamin, Doxycyclin, Edrophonium, Isoproterenol, Liothyronin, Metaraminol, Methyldopa, Minocyclin, Palcitaxel, Pentazocin, Phenylephrin, Phentolamin, Propofol, Rifamycine, Ritodrin, Taxol, Teicoplanin, Terbutalin, Tetracyclin und Vancomycin ausgewählt ist.
20. Pharmazeutische Zusammensetzung, umfassend eine wirksame Menge eines Konjugats nach irgendeiner der Ausführungsformen 12 bis 19 sowie einen pharmazeutisch annehmbaren Träger und gegebenenfalls weitere Hilfs- und Wirkstoffe.
21. Verwendung eines Konjugats nach einer der Ausführungsformen 12 bis 19 oder einer Zusammensetzung nach Ausführungsform 20 zur therapeutischen oder präventiven Behandlung von Menschen oder Tieren.
22. Verfahren zur Herstellung eines Hydroxyalkylstärke-Konjugats nach einer der Ausführungsformen 1 bis 19, **dadurch gekennzeichnet, dass** eine Kopplungsreaktion zwischen der endständigen Aldehydgruppe oder einer aus dieser Aldehydgruppe durch chemische Umsetzung hervorgegangenen funktionellen Gruppe des Hydroxyalkylstärkemoleküls und einer mit dieser Aldehydgruppe oder daraus hervorgegangenen funktionellen Gruppe des Hydroxyalkylstärkemoleküls reaktionsfähigen funktionellen Gruppe der niedermolekularen Substanz durchgeführt wird und wobei die bei der Kopplungsreaktion unmittelbar resultierende Bindung gegebenenfalls durch eine weitere Reaktion modifiziert wird.
23. Verfahren nach Ausführungsform 22, **dadurch gekennzeichnet, dass** die endständige Aldehydgruppe des Hydroxyalkylstärkemoleküls durch selektive Oxidation in die entsprechende Lactongruppe überführt und diese anschließend mit einer primären Aminogruppe der niedermolekularen Substanz umgesetzt wird, so dass das Hydroxyalkylstärkemolekül durch eine Amidbindung an die niedermolekulare Substanz gebunden wird.
24. Verfahren nach Ausführungsform 23, **dadurch gekennzeichnet, dass** die selektive Oxidation der Aldehydgruppe mit lod oder Metallionen in wässriger basischer Lösung durchgeführt wird.
25. Verfahren nach Ausführungsform 23 oder 24, **dadurch gekennzeichnet, dass** die Kopplungsreaktion in Gegenwart eines Carbodiimids, vorzugsweise 1-(3-Dimethylaminopropyl)-3-ethylcarbodiimid (EDC), durchgeführt wird.
26. Verfahren nach einer der Ausführungsformen 22 bis 25, **dadurch gekennzeichnet, dass** die Kopplungsreaktion in heterogener Phase durchgeführt wird.
27. Verfahren nach einer der Ausführungsformen 22 bis 25, **dadurch gekennzeichnet, dass** die Kopplungsreaktion in homogener Phase in DMSO oder N-Methylpyrrolidon oder Glycol durchgeführt wird.
28. Verfahren nach Ausführungsform 23 oder 24, **dadurch gekennzeichnet, dass** die Kopplungsreaktion in DMSO oder N-Methylpyrrolidon oder Glycol in Abwesenheit eines Aktivators durchgeführt wird.
29. Verfahren nach Ausführungsform 22, **dadurch gekennzeichnet, dass** die endständige Aldehydgruppe des Hydroxyalkylstärkemoleküls mit einer primären Amino- gruppe der niedermolekularen Substanz unter Bildung einer Schiffschen Base gekoppelt wird und die gebildete Schiff'sche Base zum Amin reduziert wird, so dass das Hydroxyethylstärkemolekül durch eine Aminbindung an die niedermolekulare Substanz gebunden wird.
30. Verfahren nach Ausführungsform 29, **dadurch gekennzeichnet, dass** das Reduktionsmittel Natriumborhydrid, Natriumcyanoborhydrid oder ein organischer Borkomplex ist.
31. Verfahren zur Herstellung von selektiv an der endständigen Aldehydgruppe oxidierter Hydroxyalkylstärke, **dadurch gekennzeichnet, dass** die Hydroxyalkylstärke in einem Molverhältnis von lod zu HAS von 2 : 1 bis 20 : 1 in wässriger basischer Lösung umgesetzt wird.
32. Verfahren nach Ausführungsform 31, **dadurch gekennzeichnet, dass** das Molverhältnis von lod zu HAS etwa 5 : 1 bis 6 : 1 beträgt.
33. Verfahren nach Ausführungsform 31, **dadurch gekennzeichnet, dass**
   a) eine Menge von Hydroxyalkylstärke in warmem destilliertem Wasser gelöst wird und etwas weniger als 1 Moläquivalent wässriger lodlösung zugegeben wird,
   b) NaOH-Lösung in einer molaren Konzentration, die etwa das 5-15fache der lodlösung beträgt, langsam tropfenweise in Abständen von mehreren Minuten der Reaktionslösung zugegeben wird, bis die Lösung nach der Zugabe beginnt, wieder klar zu werden,
   c) erneut etwas weniger als 1 Moläquivalent wässriger lodlösung der Reaktionslösung zugegeben wird,
   d) das Zutropfen der NaOH-Lösung wieder aufgenommen wird,
   e) die Schritte b) bis d) solange wiederholt werden, bis etwa 5,5-6 Moläquivalente lodlösung und 11-12 Moläquivalente NaOH-Lösung, bezogen auf die Hydroxyalkylstärke, zugegeben worden sind,
   f) die Reaktion dann abgebrochen und die Reaktionslösung entsalzt und einer Kationenaustauschchromatographie unterworfen und das Reaktionsprodukt durch Lyophilisierung gewonnen wird.
34. Verfahren nach Ausführungsform 33, **dadurch gekennzeichnet, dass** es sich bei der wässrigen lodlösung um eine etwa 0,05-0,5 N lodlösung handelt.
35. Verfahren nach Ausführungsform 33 oder 34, **dadurch gekennzeichnet, dass** die molare Konzentration der NaOH-Lösung etwa das 10fache der lodlösung beträgt.
36. Verfahren zur Herstellung von selektiv an der endständigen Aldehydgruppe oxidierter Hydroxyalkylstärke, **dadurch gekennzeichnet, dass** die HAS in wässriger alkalischer Lösung mit einem molaren Überschuss an stabilisierten Metallionen, ausgewählt aus Cu2+-lonen und Ag+-lonen, oxidiert wird.

## Patentansprüche

1. Konjugat von Hydroxyalkylstärke und einer niedermolekularen Substanz, **dadurch gekennzeichnet, dass** die bindende Wechselwirkung zwischen dem Hydroxyalkylstärkemolekül und der niedermolekularen Substanz auf einer kovalenten Bindung beruht, welche das Ergebnis einer Kopplungsreaktion
(a) zwischen (i) der endständigen Aldehydgruppe oder einer aus dieser Aldehydgruppe durch chemische Umsetzung hervorgegangenen funktionellen Gruppe des Hydroxyalkylstärkemoleküls und (ii) einer mit dieser Aldehydgruppe oder daraus hervorgegangenen funktionellen Gruppe des Hydroxyalkylstärkemoleküls reaktionsfähigen funktionellen Gruppe der niedermolekularen Substanz ist, wobei die reaktionsfähige funktionelle Gruppe der niedermolekularen Substanz eine der funktionellen Gruppen eines bifunktionellen Linkermoleküls ist, welches an die niedermolekulare Substanz gekoppelt wurde, oder
(b) zwischen (i) einer aus der endständigen Aldehydgruppe des Hydroxyalkylstärkemoleküls durch chemische Umsetzung hervorgegangenen funktionellen Gruppe des Hydroxyalkylstärkemoleküls und (ii) einer mit dieser aus der chemischen Umsetzung hervorgegangenen funktionellen Gruppe des Hydroxyalkylstärkemoleküls reaktionsfähigen funktionellen Gruppe der niedermolekularen Substanz ist, wobei die aus der endständigen Aldehydgruppe des Hydroxyalkylstärkemoleküls durch chemische Umsetzung hervorgegangene funktionelle Gruppe eine der funktionellen Gruppen eines bifunktionellen Linkermoleküls ist, mit dem die endständige Aldehydgruppe oder eine daraus hervorgegangene funktionelle Gruppe umgesetzt wurde,
wobei die bei der Kopplungsreaktion unmittelbar resultierende Bindung gegebenenfalls durch eine weitere Reaktion zur obengenannten kovalenten Bindung modifiziert sein kann.

2. Konjugat nach Anspruch 1, **dadurch gekennzeichnet, dass** die aus der endständigen Aldehydgruppe des Hydroxyalkylstärkemoleküls durch chemische Umsetzung hervorgegangene funktionelle Gruppe in (a) eine der funktionellen Gruppen eines bifunktionellen Linkermoleküls ist, mit dem die endständige Aldehydgruppe oder eine daraus hervorgegangene funktionelle Gruppe umgesetzt wurde.

3. Konjugat nach Anspruch 1 oder 2, **dadurch gekennzeichnet, dass** die reaktionsfähige funktionelle Gruppe der niedermolekularen Substanz in (b) eine der funktionellen Gruppen eines bifunktionellen Linkermoleküls ist, welches an die niedermolekulare Substanz gekoppelt wurde.

4. Konjugat nach einem der Ansprüche 1 bis 3, **dadurch gekennzeichnet, dass** in (a) die kovalente Bindung das Ergebnis einer Kopplungsreaktion zwischen einer durch selektive Oxidation der endständigen Aldehydgruppe des Hydroxyalkylstärkemoleküls gebildeten Carboxylgruppe oder aktivierten Carboxylgruppe und einer primären Aminogruppe oder Thiolgruppe der niedermolekularen Substanz ist.

5. Konjugat nach Anspruch 4, **dadurch gekennzeichnet, dass** die kovalente Bindung eine Amidbindung ist, welche das Ergebnis einer Kopplungsreaktion zwischen einem durch selektive Oxidation der endständigen Aldehydgruppe des Hydroxyalkylstärkemoleküls gebildeten Lacton und einer primären Aminogruppe der niedermolekularen Substanz ist.

6. Konjugat nach einem der Ansprüche 1 bis 3, **dadurch gekennzeichnet, dass** in (a) die kovalente Bindung eine Aminbindung ist, welche das Ergebnis einer Kopplungsreaktion zwischen der endständigen Aldehydgruppe des Hydroxyalkylstärkemoleküls und einer primären Aminogruppe der niedermolekularen Substanz unter Bildung einer Schiff'schen Base und der Reduktion der Schiff'schen Base zum Amin ist.

7. Konjugat nach einem der Ansprüche 1 bis 6, **dadurch gekennzeichnet, dass** das Hydroxyalkylstärkemolekül ein Molekulargewicht im Bereich von etwa 70 bis etwa 1000 kD aufweist.

8. Konjugat nach einem der Ansprüche 1 bis 7, **dadurch gekennzeichnet, dass** das Hydroxyalkylstärkemolekül einen Substitutionsgrad von etwa 0,3 bis etwa 0,7 aufweist.

9. Konjugat nach einem der Ansprüche 1 bis 8, **dadurch gekennzeichnet, dass** das Hydroxyalkylstärkemolekül ein Verhältnis der C2-zu C6-Substitution von 8 bis 12 aufweist.

10. Konjugat nach einem der Ansprüche 1 bis 9, **dadurch gekennzeichnet, dass** das Hydroxyalkylstärkemolekül ein Hydroxyethylstärkemolekül ist.

11. Konjugat nach einem der Ansprüche 1 bis 10, **dadurch gekennzeichnet, dass** die niedermolekulare Substanz ein Arzneiwirkstoff ist.

12. Konjugat nach Anspruch 11, **dadurch gekennzeichnet, dass** der Arzneiwirkstoff aus der Gruppe aus Antibiotika, Antidepressiva, Antidiabetika, Antidiuretika, Anticholinergika, Antiarrhytmika, Antiemetika, Antiepileptika, Antihistaminika, Antimykotika, Antisympathotonika, Antithrombotika, Androgenen, Antiandrogenen, Estrogenen, Antiestrogenen, Antiosteoporosemitteln, Antitumormitteln, Vasodilatatoren und anderen blutdrucksenkenden Mitteln, fiebersenkenden Mitteln, Schmerzmitteln, entzündungshemmenden Mitteln, beta-Blockern, Immunsuppressiva und Vitaminen ausgewählt ist.

13. Konjugat nach Anspruch 11 oder 12, **dadurch gekennzeichnet, dass** die an der Kopplungsreaktion beteiligte funktionelle Gruppe des Arzneiwirkstoffs eine Aminogruppe ist.

14. Konjugat nach Anspruch 13, **dadurch gekennzeichnet, dass** der Arzneiwirkstoff aus der Gruppe aus Albuterol, Alendronat, Amikazin, Aminopenicillin, Amoxicillin, Atenolol, Azathioprin, Cefaclor, Cefadroxil, Cefotaxim, Ceftazidim, Ceftriaxon, Cilastatin, Cimetidin, Ciprofloxacin, Clonidin, Colistin, Cosyntropin, Cycloserin, Daunorubicin, Doxorubicin, Desmopressin, Dihydroergotamin, Dobutamin, Dopamin, Ephedrin, Epinephrin, epsilon-Aminocapronsäure, Ergometrin, Esmolol, Famotidin, Flecainid, Folsäure, Flucytosin, Furosemid, Ganciclovir, Gentamicin, Glucagon, Hydrazalin, Imipenem, Isoproterenol, Ketamin, Liothyronin, Lutiliberin, Merpatricin, Metaraminol, Methyldopa, Metoclopramid, Metoprolol, Mexiletin, Mitomycin, Neomycin, Netilmicin, Nimodipin, Nystatin, Octreotid, Oxytocin, Pamidronat, Pentamidin, Phentolamin, Phenylephrin, Procainamid, Procain, Propranolol, Ritodrin, Sotalol, Teicoplanin, Terbutalin, Thiamin, Tiludronat, Tolazolin, Trimethoprim, Tromethamin, Vancomycin, Vasopressin und Vinblastin ausgewählt ist.

15. Konjugat nach Anspruch 11 oder 12, **dadurch gekennzeichnet, dass** die an der Kopplungsreaktion beteiligte funktionelle Gruppe des Arzneiwirkstoffs eine Carboxylgruppe oder aktivierte Carboxylgruppe ist.

16. Konjugat nach Anspruch 15, **dadurch gekennzeichnet, dass** der Arzneiwirkstoff aus der Gruppe aus Acetylcystein, Azlocillin, Aztreonam, Benzylpenicillin, Camptothecin, Cefamandol, Cefazolin, Cefepim, Cefotaxim, Cefotetan, Cefoxitin, Ceftazidim, Ceftriaxon, Cephalothin, Cilastatin, Ciprofloxacin, Clavulansäure, Dicloxacillin, epsilon-Aminocapronsäure, Floxacillin, Folinsäure, Furosemid, Fusidinsäure, Imipemem, Indomethacin, Ketorolac, Liothyronin, Melphalan, Methyldopa, Piperacillin, Prostacyclin, Prostaglandine, Teicoplanin, Ticarcillin und Vancomycin ausgewählt ist.

17. Konjugat nach Anspruch 11 oder 12, **dadurch gekennzeichnet, dass** die an der Kopplungsreaktion beteiligte funktionelle Gruppe des Arzneiwirkstoffs eine aliphatische oder Aryl-OH-Gruppe ist.

18. Konjugat nach Anspruch 17, **dadurch gekennzeichnet, dass** der Arzneimittelwirkstoff aus der Gruppe aus Albuterol, Allopurinol, Apomorphin, Ceftriaxon, Dobutamin, Dopamin, Doxycyclin, Edrophonium, Isoproterenol, Liothyronin, Metaraminol, Methyldopa, Minocyclin, Paclitaxel, Pentazocin, Phenylephrin, Phentolamin, Propofol, Rifamycine, Ritodrin, Taxol, Teicoplanin, Terbutalin, Tetracyclin und Vancomycin ausgewählt ist.

19. Pharmazeutische Zusammensetzung, umfassend eine wirksame Menge eines Konjugats nach irgendeinem der Ansprüche 11 bis 18 sowie einen pharmazeutisch annehmbaren Träger und gegebenenfalls weitere Hilfs- und Wirkstoffe.

20. Verwendung eines Konjugats nach einem der Ansprüche 11 bis 18 oder einer Zusammensetzung nach Anspruch 19 zur therapeutischen oder präventiven Behandlung von Menschen oder Tieren.

21. Verfahren zur Herstellung eines Hydroxyalkylstärke-Konjugats nach einem der Ansprüche 1 bis 18, **dadurch gekennzeichnet, dass** eine Kopplungsreaktion durchgeführt wird
(a) zwischen (i) der endständigen Aldehydgruppe oder einer aus dieser Aldehydgruppe durch chemische Umsetzung hervorgegangenen funktionellen Gruppe des Hydroxyalkylstärkemoleküls und einer mit dieser Aldehydgruppe oder daraus hervorgegangenen funktionellen Gruppe des Hydroxyalkylstärkemoleküls reaktionsfähigen funktionellen Gruppe der niedermolekularen Substanz, wobei die reaktionsfähige funktionelle Gruppe der niedermolekularen Substanz eine der funktionellen Gruppen eines bifunktionellen Linkermoleküls ist, welches an die niedermolekulare Substanz gekoppelt wurde,
oder
(b) zwischen (i) einer aus der endständigen Aldehydgruppe des Hydroxyalkylstärkemoleküls durch chemische Umsetzung hervorgegangenen funktionellen Gruppe des Hydroxyalkylstärkemoleküls und (ii) einer mit dieser aus der Umsetzung hervorgegangenen funktionellen Gruppe des Hydroxyalkylstärkemoleküls reaktionsfähigen funktionellen Gruppe der niedermolekularen Substanz, wobei die aus der endständigen Aldehydgruppe des Hydroxyalkylstärkemoleküls durch chemische Umsetzung hervorgegangene funktionelle Gruppe eine der funktionellen Gruppen eines bifunktionalen Linkermoleküls ist, mit dem die endständige Aldehydgruppe oder eine daraus hervorgegangene funktionelle Gruppe umgesetzt wurde,
und wobei die bei der Kopplungsreaktion unmittelbar resultierende Bindung gegebenenfalls durch eine weitere Reaktion modifiziert wird.

22. Verfahren nach Anspruch 21, **dadurch gekennzeichnet, dass** in (a) die endständige Aldehydgruppe des Hydroxyalkylstärkemoleküls durch selektive Oxidation in die entsprechende Lactongruppe überführt und diese anschließend mit einer primären Aminogruppe der niedermolekularen Substanz umgesetzt wird, so dass das Hydroxyalkylstärkemolekül durch eine Amidbindung an die niedermolekulare Substanz gebunden wird, wobei die selektive Oxidation der Aldehydgruppe bevorzugt mit lod oder Metallionen in wässriger basischer Lösung durchgeführt wird.

23. Verfahren nach Anspruch 22, **dadurch gekennzeichnet, dass** die Kopplungsreaktion in Gegenwart eines Carbodiimids, vorzugsweise 1-(3-Dimethylaminopropyl)-3-ethylcarbodiimid (EDC), durchgeführt wird.

24. Verfahren nach Anspruch 21, **dadurch gekennzeichnet, dass** in (a) die endständige Aldehydgruppe des Hydroxyalkylstärkemoleküls mit einer primären Aminogruppe der niedermolekularen Substanz unter Bildung einer Schiff'schen Base gekoppelt wird und die gebildete Schiff'sche Base zum Amin reduziert wird, so dass das Hydroxyethylstärkemolekül durch eine Aminbindung an die niedermolekulare Substanz gebunden wird, wobei das Reduktionsmittel bevorzugt Natriumborhydrid, Natriumcyanoborhydrid oder ein organischer Borkomplex ist.

25. Verfahren zur Herstellung von selektiv an der endständigen Aldehydgruppe oxidierter Hydroxyalkylstärke, **dadurch gekennzeichnet, dass** die Hydroxyalkylstärke in einem Molverhältnis von lod zu HAS von 2 : 1 bis 20 : 1 in wässriger basischer Lösung umgesetzt wird, wobei das Molverhältnis von lod zu HAS bevorzugt etwa 5 : 1 bis 6 : 1 beträgt, und wobei das Verfahren bevorzugt **dadurch gekennzeichnet ist, dass**
a) eine Menge von Hydroxyalkylstärke in warmem destilliertem Wasser gelöst wird und etwas weniger als 1 Moläquivalent wässriger lodlösung zugegeben wird,
b) NaOH-Lösung in einer molaren Konzentration, die etwa das 5-15fache der lodlösung beträgt, langsam tropfenweise in Abständen von mehreren Minuten der Reaktionslösung zugegeben wird, bis die Lösung nach der Zugabe beginnt, wieder klar zu werden,
c) erneut etwas weniger als 1 Moläquivalent wässriger lodlösung der Reaktionslösung zugegeben wird,
d) das Zutropfen der NaOH-Lösung wieder aufgenommen wird,
e) die Schritte b) bis d) solange wiederholt werden, bis etwa 5,5-6 Moläquivalente lodlösung und 11-12 Moläquivalente NaOH-Lösung, bezogen auf die Hydroxyalkylstärke, zugegeben worden sind,
f) die Reaktion dann abgebrochen und die Reaktionslösung entsalzt und einer Kationenaustauschchromatographie unterworfen und das Reaktionsprodukt durch Lyophilisierung gewonnen wird.

26. Verfahren zur Herstellung von selektiv an der endständigen Aldehydgruppe oxidierter Hydroxyalkylstärke, **dadurch gekennzeichnet, dass** die HAS in wässriger alkalischer Lösung mit einem molaren Überschuss an stabilisierten Metallionen, ausgewählt aus Cu²⁺-lonen und Ag⁺-lonen, oxidiert wird.
